# EUROPEAN PATENT APPLICATION

(11) **EP 4 748 290 A1**
(43) Date of publication of application: **27.05.2026**
(21) Application number: 25211820.3
(22) Date of filing: 28.10.2025
(51) Int. Cl.: A61B 1/00

(54) **A NOVEL POLYP TRAP**

(30) Priority: 25.11.2024 US 202418958527
(71) Applicant: GA HEALTH COMPANY LIMITED, Shatin, N.T. (HK)
(72) Inventor: CHAN, Tsan Ho, Hong Kong (CN)
(74) Representative: Ullrich & Naumann PartG mbB

(57) **Abstract**

The present invention is related to a polyp trap, configurable to connect to an endoscope and a vacuum system in endoscopy procedures, comprising: a hollow main body having an inlet and an outlet, wherein said main body, said inlet, and said outlet are fluidically communicable; and a tube having a first end removably connectable to said inlet and a second end removably connectable to the endoscope, characterized in that, said polyp trap further comprising a first stand positioned peripherally to said main body for receiving said second end of said tube, whereby said tube can selectively connect to said first stand when said polyp trap is in packaging or connect to the endoscope when said polyp trap is in the endoscopy procedures.

## Description

### FIELD OF THE INVENTION

The present invention is related to a specialized device designed to capture and retrieve polyps or tissue samples removed during endoscopy. To be more specific, the present invention is related to a polyp trap which is used during endoscopy to capture and retrieve polyps or tissue samples removed during the endoscopy procedure.

### BACKGROUND OF THE INVENTION

An endoscopy is a medical procedure that involves using a long, flexible tube with a camera and light on the end, known as an endoscope, to examine the interior of a person's body. This procedure allows healthcare providers to visualize and evaluate various organs and structures without the need for invasive surgery. Endoscopies can be performed for different purposes, such as upper endoscopy (EGD): Examines the esophagus, stomach, and the upper part of the small intestine; colonoscopy: Examines the colon and rectum; bronchoscopy: Examines the airways in the lungs; capsule endoscopy: Involves swallowing a small, pill-sized camera to visualize the small intestine; and endoscopic ultrasound (EUS): Combines endoscopy with ultrasound to examine organs like the pancreas and bile ducts. During an endoscopy, the endoscope is carefully inserted through a natural opening in the body, such as the mouth or rectum. The camera at the tip of the endoscope captures images of the internal structures, which are displayed on a monitor for the healthcare provider to view in real-time. Endoscopies are commonly used for diagnostic purposes to investigate symptoms like abdominal pain, gastrointestinal bleeding, or difficulty swallowing. They can also be used for therapeutic interventions, such as removing polyps, taking tissue samples for biopsy, or treating certain conditions.

When conducting endoscopy, say, a colonoscopy, if a polyp is detected, it is common practice to remove it for further examination or to prevent potential complications. The polyp trap serves as a container or mechanism within the endoscope that securely holds and collects the polyps or tissue samples as they are snared or excised by the endoscopist. This allows for the safe retrieval of the polyps without losing them within the colon, ensuring that they can be sent to a pathology lab for analysis. The use of a polyp trap is a standard practice in modern endoscopy to facilitate the efficient and safe removal of polyps or tissue samples during colonoscopies and other procedures where tissue samples need to be collected for diagnostic purposes.

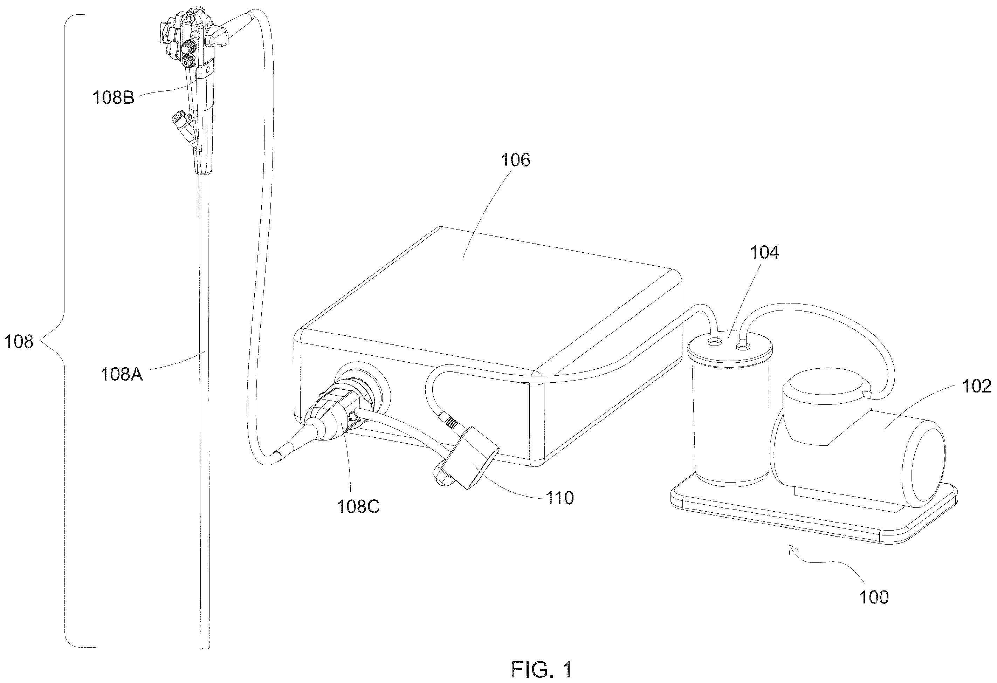

Figure 1 illustrate a state of art set-up 100 for performing endoscopy. The set-up 100 comprises a suction pump 102, a collection tank 104, light source 106, an endoscope 108 including an insertion section 108A, control section 108B, and a connector section 108C, and a polyp trap 110 which is essential for capturing and sieving polyps and/or tissue samples removed during endoscopy procedures. These components are fluidically connected as illustrated in figure 1, which are commonly known by those skilled in the art, thus the connection and working/operational mechanism of these components are not described in details for clarity's sake. For clarity's sake, an imaging management system typically used in endoscopy procedures is not included in the drawings.

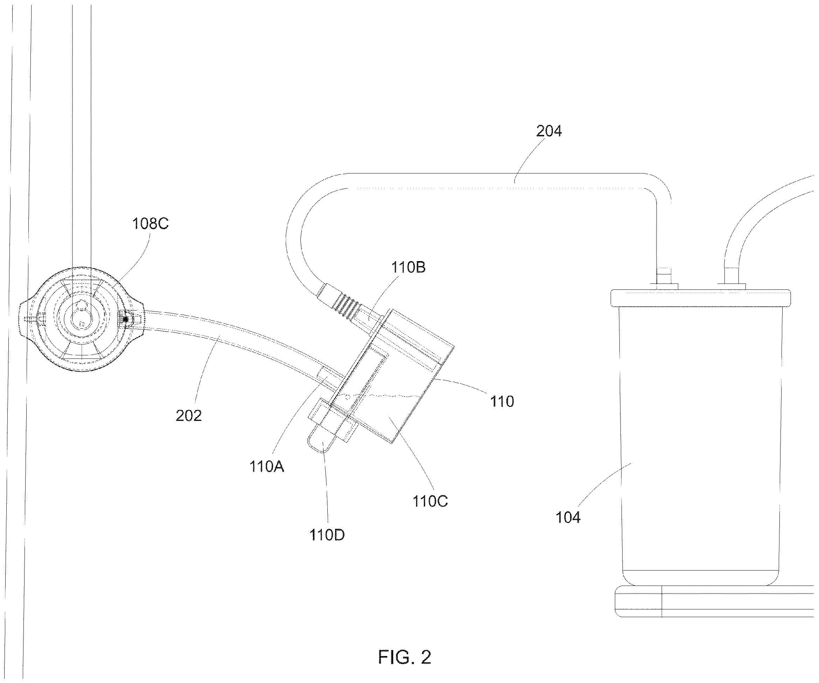

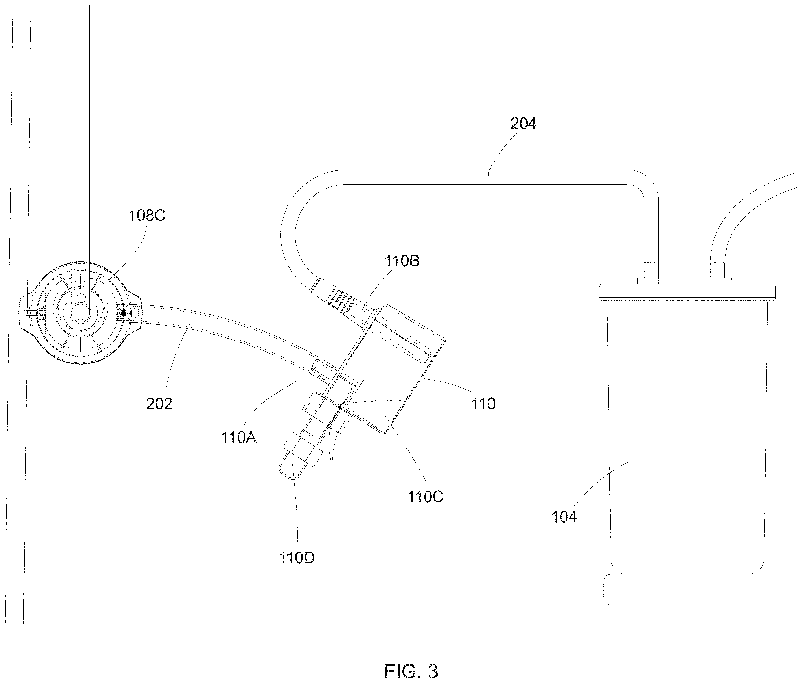

Figure 2 is a close-up view showing the condition and orientation of the polyp trap 110 during the endoscopy procedure. As seen, the polyp trap 110 comprises an inlet 110A connecting to the connector section 108C of the endoscope 108 via tube 202 and an outlet 110B connecting to the collection tank 104 via tube 204. Tube 202 and 204 are commonly made of silicone, polypropylene, or rubber materials known for their flexibility, non-toxic properties, and biocompatibility, making them ideal for medical applications like endoscopy. However, the inherent flexibility of the tube materials always causes the tube to maintain a straight profile, which in turn results in the tilting of the polyp trap during endoscopic procedures. In Figure 2, the illustration demonstrates the tilted orientation of the polyp trap 110, where the outlet 110B sits at a higher position than the inlet 110A. This positioning causes the tissue fluid or liquid 110C extracted from the body to accumulate or pool near or at the door 110D of the polyp trap 110. As a result, the collected material may not flow smoothly out of the polyp trap 110 towards the collection tank 104, potentially affecting the efficiency of the endoscopy procedures since the suction is hindered. Moreover, a critical issue arises when a medical professional, responsible for conducting endoscopy procedures, opens the door 110D of the polyp trap 110 to extract polyps or tissue samples. At this point, the accumulated tissue fluid or liquid 110C within the polyp trap 110 is prone to splashing, as depicted in figure 3. This scenario presents a significant challenge as the potential splashing of the collected material not only poses a risk to the operator but also compromises the cleanliness and sterility of the procedure. Such splashing can lead to contamination risks and may hinder the smooth progression of the endoscopy process, impacting the overall effectiveness of the medical intervention.

The present invention at least seeks to address issues of these problems, or at least to provide an alternative to the public.

### SUMMARY OF THE INVENTION

The first aspect of the present invention is related to a polyp trap, configurable to connect to an endoscope and a vacuum system in endoscopy procedures, comprising: a hollow main body having an inlet and an outlet, wherein said main body, said inlet, and said outlet are fluidically communicable; and a tube having a first end removably connectable to said inlet and a second end removably connectable to the endoscope, characterized in that, said polyp trap further comprising a first stand positioned peripherally to said main body for receiving said second end of said tube, whereby said tube can selectively connect to said first stand when said polyp trap is in packaging or connect to the endoscope when said polyp trap is in the endoscopy procedures.

In some embodiments, said main body comprising an opening providing access to the internal cavity of said main body; and said polyp trap comprising a door having a first end hingedly connected to said main body and a second end profiled as a handle, whereby said door is configured to open and close said opening with said handle by flipping down vertically and flipping up vertically, respectively. More preferably, said door being provided with a circumferential wall on one of its surfaces, wherein said circumferential wall is sized and shaped to be fit snugly within said opening of said main body. Most preferably, surrounding the perimeter of said circumferential wall is at least one sealing ridge for enhancing the sealing capability of said door when closing said opening. Advantageously, said sealing ridge is made of resilient material.

In some embodiments, the polyp trap in the first aspect further comprising at least one second stand positioned peripherally to said main body, and at least one pick removably received by said second stand; and/or a tray received in said main body and designed as a receptacle having a circumferential wall and a perforated bottom, wherein said perforated bottom being configured as a concave shape with the curved surface facing towards said inlet; and/or at least one stopper standing inside said main body. Preferably, said main body is made of transparent material and/or said main body comprising an opening providing access to the internal cavity of said main body; and said polyp trap comprising a door having a first end hingedly connected to said main body and a second end profiled as a handle, whereby said door is configured to open and close said opening with said handle by flipping down vertically and flipping up vertically, respectively. More preferably, said door being provided with a circumferential wall on one of its surfaces, wherein said circumferential wall is sized and shaped to be fit snugly within said opening of said main body. Most preferably, surrounding the perimeter of said circumferential wall is at least one sealing ridge for enhancing the sealing capability of said door when closing said opening. Advantageously, said sealing ridge is made of resilient material.

The second aspect of the present invention is related to the use of the polyp trap of the first aspect for endoscopy procedures, which ensures that the fluid inside the polyp trap remains at a consistent level and can be efficiently removed during the endoscopy procedures.

### DESCRIPTION OF THE DRAWINGS

Some embodiments of the present invention will now be explained, with reference to the accompanied drawings, in which:-
- Figure 1: is perspective view showing a general set-up for performing endoscopy in which a state of art polyp trap is used;
- Figure 2: is a close-up view showing the condition of the polyp trap during the endoscopy procedures;
- Figure 3: depicts the accumulated tissue fluid or liquid within the polyp trap is prone to splashing;
- Figures 4A-4D: depict a polyp trap 310 of the present invention from different views;
- Figures 5A-5C: depict the orientation of the polyp trap 310 when in use from different views;
- Figures 6A-6B: depict the use of a pick of the present invention to remove a collected polyp from a tray of the present invention from different views; and
- Figures 7A-7B: depict the use of a pick to remove a collected polyp from a tray in the state of art from different views.

### DETAILED DESCRIPTION OF PREFERRED EMBODIMENTS OF THE INVENTION

The present invention is now presented by way of examples with reference to the figures in the following paragraphs. Objects, features, and aspects of the present disclosure are disclosed in or are apparent from the following description. It should be understood by one of ordinary skilled in the art that the following description is a description of exemplary embodiments only, and is not intended as limiting the broader aspects of the present disclosure, which broader aspects are embodied in the exemplary constructions.

It should be noted that, unless otherwise defined, the technical terms or scientific terms used in the embodiments of the present invention shall have the usual meanings understood by person with ordinary skills in the art to which the present invention belongs. "First", "second" and similar expression used in the embodiments of the present invention do not indicate any order, quantity or importance, but are only used to distinguish different components. "Front", "rear", "left", "right", "upper", and "lower" and other terms indicating orientation or similar terms are only described for the exemplary relative positional relationship shown in the drawings to facilitate the understanding. It does not limit the disclosed components in the present invention can only follow this specific relative positional relationship.

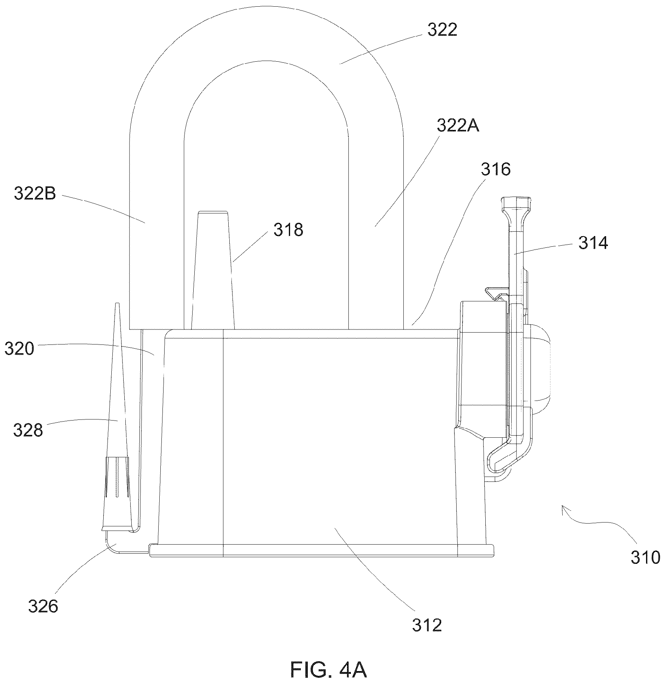

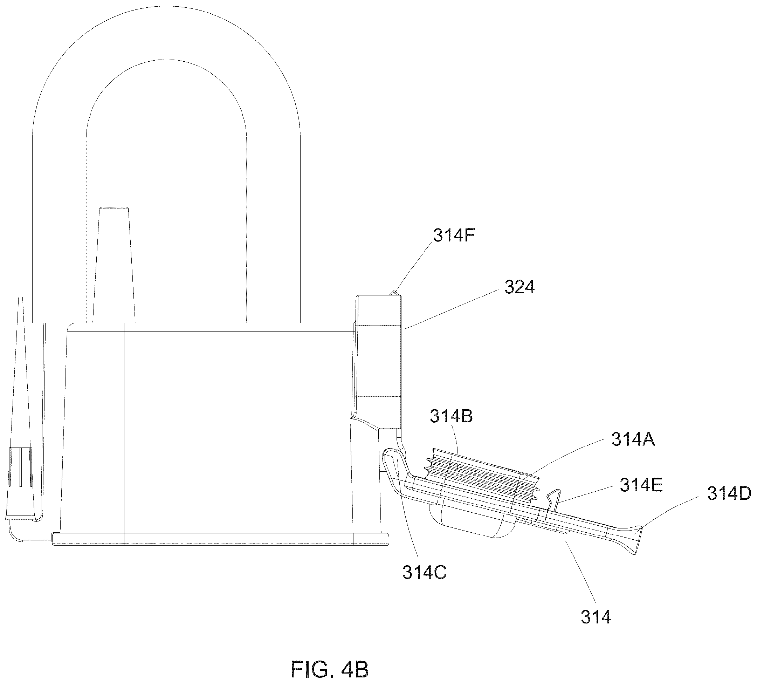

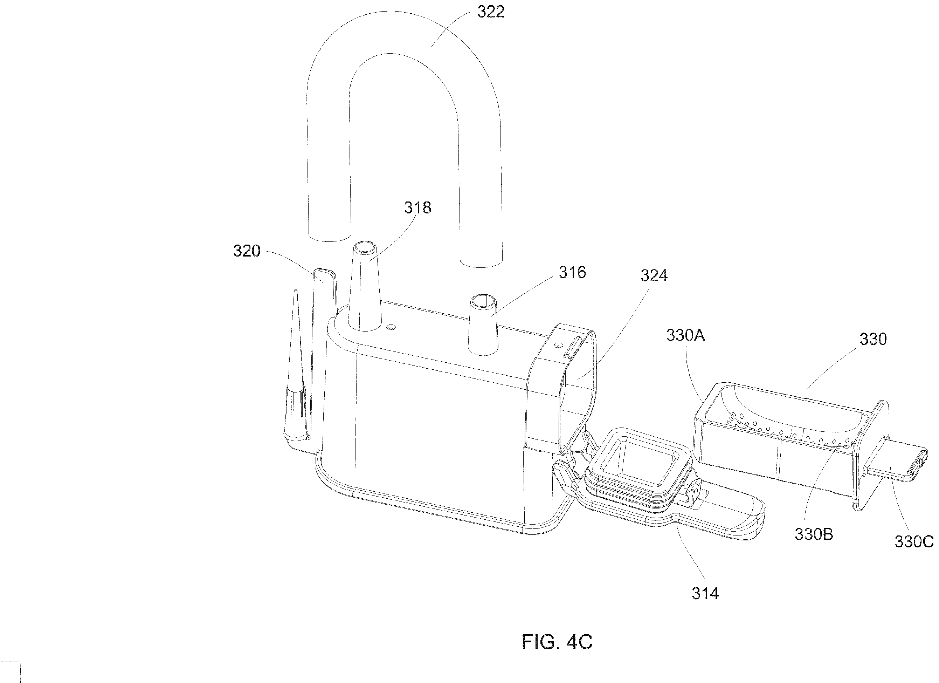

Figures 4A-4D depict a polyp trap 310 of the present invention from different views. Figure 4A is a side view depicting the polyp trap 310, which comprises a main body 312 which is hollow. The main body 312 is provided with an opening 324 (c.f. figure 4B) providing access to the internal cavity of the polyp trap, an inlet 316 (c.f. figures 4C-4D) and an outlet 318 both provided on the circumferential wall of the main body. Advantageously, the main body, the inlet, and the outlet are fluidically communicable. More advantageously, the main body, the opening, the inlet, and the outlet are fluidically communicable.

The polyp trap 310 comprises a door 314 which is in closed condition for closing the opening 324, a first stand 320 positioned peripherally to the main body 312, a tube 322 having a first end 322A arranged to removably connect to the inlet 316 and a second end 322B arranged to removably connect to the first stand 320.

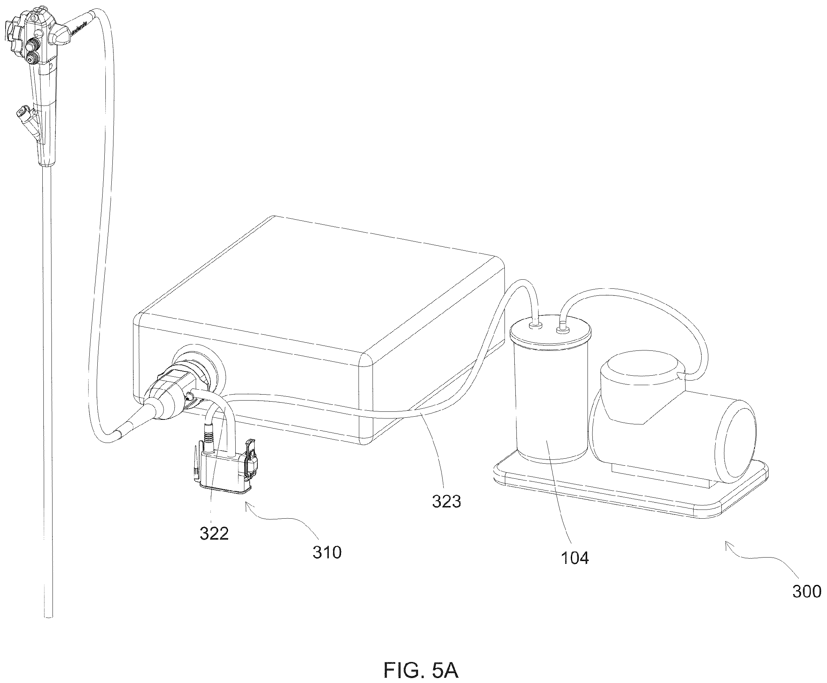

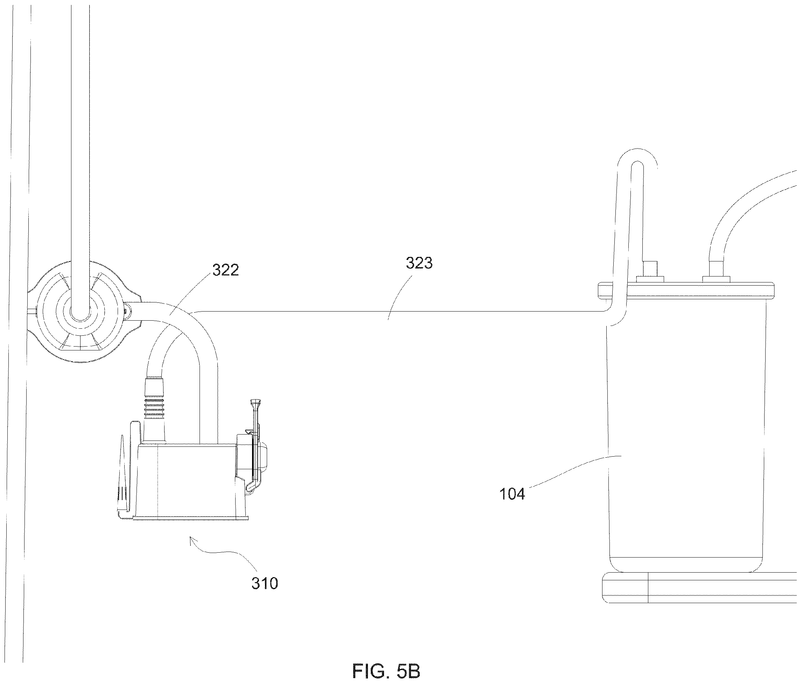

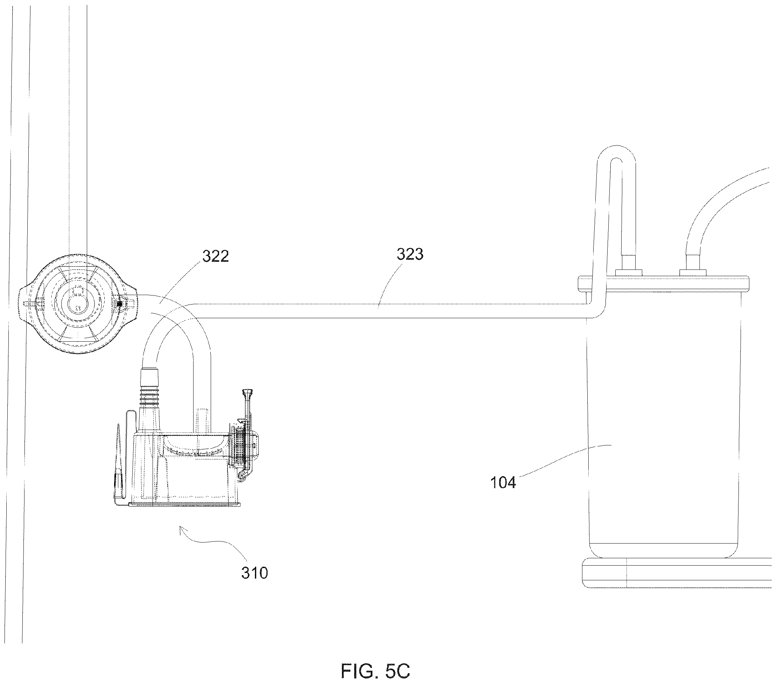

It is important to note that the second end 322B of the tube 322 is arranged to connect to the first stand 320 when the polyp trap 310 is in packaging. It is further important to note that when the endoscopy procedure is being performed, i.e., when the polyp trap 310 is in use, the second end 322B of the tube 322 is arranged to connect to the connector section 108C of the endoscope 108. Advantageously, the main body 312 is made of transparent material, enhancing visibility allowing faster identification and response to collected polyps and/or tissue samples. It shall be understood that while the door 314 is closed, the polyp trap 310 is in a closed configuration. It shall also be understood that the inlet in a polyp trap is the point where materials such as tissue samples, polyps, or fluids enter the trap. During an endoscopic procedure, when polyps or tissue samples are removed, they are directed into the polyp trap through the inlet. The inlet is designed to allow easy entry of these materials into the trap for collection and isolation. It shall also be understood that the outlet in a polyp trap is where fluids or materials exit the trap after being collected or separated. In the context of a polyp trap, the outlet is where fluids drain out, leaving behind the collected tissue samples or polyps. This allows for the efficient removal of fluids while retaining the solid materials for further examination or analysis. The first stand 320 is sized and shaped to be received within the second end 322B of the tube 322. In some embodiments, the first stand 320 is designed to provide a clip for an example to receive the second end 322B. The purpose of the first stand 320 is to maintain the tube 322 in a bent profile, especially when the polyp trap is in packaging before use. This practice is highly advantageous and is explained in figures 5A-5C which depict a set-up 300 for performing endoscopy, in which the polyp trap 310 is in use in which the tube 322 connects the inlet of the polyp trap to the connector section of the endoscopy and the tube 323 connects the outlet of the polyp trap to the collection tank 104. As seen, the polyp trap 310 in figures 5A-5C is kept in a parallel orientation or horizontal orientation due to the tube 322 being held in a bent profile, mitigating the problem illustrated in figure 2 and discussed in the BACKGROUND OF THE INVENTION. This addresses the issue arising from the inherent flexibility of tube materials, which typically causes the tube to maintain a straight profile, leading to the tilting of the polyp trap during endoscopic procedures. As depicted in figure 5C, the fluid or liquid inside the main body 312 is maintained at a consistent level. Moreover, the fluid or liquid inside the main body 312 can be effectively sucked away towards the collection tank 104.

Although the advantages of a bent tube have been recognized in the art, current practices often resort to using either auxiliary clips to maintain the tube's bent shape during use or special packaging to preserve its curvature while in storage. However, these solutions come with their own set of challenges and disadvantages. The use of auxiliary clips, while intended to support the bent profile of the tube, can introduce inconveniences for medical personnel conducting endoscopic procedures. The need to manage and adjust these clips during delicate operations may impede the efficiency and precision of the medical process. On the other hand, the reliance on special packaging designs to keep the tube bent in storage can lead to logistical issues. The bulkiness of such packaging occupies significant space, posing challenges in storage and transportation, particularly in environments where space is limited. Moreover, both these approaches contribute to increased material consumption. Whether through the use of auxiliary clips or specialized packaging, additional resources are required, thereby elevating production costs. This uptick in material usage not only impacts product expenses but also raises environmental concerns. The excessive consumption of materials can lead to higher waste generation, potentially harming the environment through resource depletion and waste management challenges.

On the contrary, the present invention offers a distinct advantage. Through its unique design, this innovation enables the tube to retain its bent shape without the need for excessive materials or elaborate packaging. By leveraging this special design feature, the invention not only ensures the tube remains in the desired bent profile but also achieves this objective with efficiency and sustainability in mind. This streamlined approach minimizes the reliance on additional materials and intricate packaging solutions, distinguishing it from conventional practices that tend to increase resource consumption and product costs. The inventive design not only simplifies the process of maintaining the tube's shape but also addresses the challenges posed by existing methods. Medical professionals can benefit from a more user-friendly and straightforward solution that enhances the efficiency of endoscopic procedures without introducing unnecessary complexities. Furthermore, by reducing material usage and optimizing packaging requirements, the invention aligns with principles of environmental responsibility. By minimizing waste generation and resource consumption, it contributes to a more sustainable approach that is both cost-effective and eco-friendly.

Figure 4B is a side view depicting the main body 312 with the door 314 in an open position, revealing the opening 324 of the main body 312. It can be seen that the door 314 is designed with a planar shape that includes a first plane outwardly accessible to a user and a second plane opposite the first plane. The second plane is provided with circumferential wall 314A at the central region thereof, which is sized and shaped to be fit snugly within the opening 324 of the main body 312. It shall be understood that while the door 314 is open, the polyp trap 310 is in an open configuration. Advantageously, the door 314 is hingedly connected to one of the sides of the main body 312 of the polyp trap 310, preferably on a side adjacent to the inlet of the polyp trap, and is designed with a first end 314C hingedly connected to the main body 312 and a second end that features an elongated handle 314D. During endoscopy procedures, the internal cavity of the main body is in negative pressure condition, therefore it is advantageous to design the door to be opened and closed by flipping down vertically and flipping up vertically, respectively, using the handle 314D. Additionally, such an opening and closing arrangement offers a best view angle for the medical personnel to observe the polyp and/or tissue sample collected by the polyp trap. More advantageously, the door 314 is provided with a tongue 314E which is engageable with a protrusion 314F provided on the circumferential wall of the main body 312 to securely close the door 314, preventing accidental opening of the door 314.

Surrounding the perimeter of the circumferential wall 314A is at least one sealing ridge 314B, which enhance the sealing capability of the door when closed. Advantageously, there are multiple sealing ridges 314B. More advantageously, said ridge is made of resilient material such as silicone. It shall be understood that the better sealing capability provides better negative pressure environment during endoscopy, which is vital for clear visualization, reducing interference, improving control, enhancing safety, and providing optimal conditions for the medical staff. By removing air and obstructions, a negative pressure environment ensures a clearer view of internal structures, minimizes distortion in images, allows for precise maneuvering of the endoscope, reduces the risk of complications like perforations, and creates the ideal setting for delicate procedures, ultimately contributing to the successful and safe execution of endoscopic examinations.

Referring to figure 4A again, the polyp trap 310 further comprises at least one second stand 326, and at least one pick 328 adapted to be installed over the second stand 326. Advantageously, in some embodiments, the second stand is positioned peripherally to the main body of the polyp trap 310. More advantageously, the size of the second stand 326 is dimensioned fit to mate with the internal diameter of the pick 328, such that medical personnel can pick up the pick 328 easily during endoscopy procedures. The purpose of the pick 328 is for the medical staff to remove the collected polyps or/and tissue samples, which is/are trapped in the polyp trap 310. Preferably, the pick 328 is made of flexible material. Preferably, the second stand 326 extends upwards from the bottom of the main body 312. However, it shall be understood that the demonstration for the second stand in figures is an example only. The spirit of the present invention resides in that the polyp trap is equipped with a stand for receiving the pick 328. In some embodiments, the number of the second stand can be two, three, four, or any integer. Preferably, the number of pick is the same as the number of second stand.

Figures 4C-4D is a perspective view depicting the polyp trap 310 in which the door 314 is opened, and a tray 330 of the polyp trap 310 is removed from its hollow main body. It can be seen that the first stand 320 extends upwards from the bottom of the main body 312. In some embodiments, the first stand is positioned peripherally to the main body of the polyp trap. However, it shall be understood that the demonstration for the first stand in figures is an example only. The spirit of the present invention resides in that the polyp trap is equipped with a stand for receiving the second end 322B of the tube 322. Advantageously, the tray 330 is made of transparent material. The purpose of the tray 330 is to collect the polyps or/and tissue samples removed during the endoscopy procedures. The tray 330 is dimensioned fit to be received in the opening 324 and is designed as a receptacle having a circumferential wall 330A, a perforated bottom 330B, and a handle 330C. The perforated bottom 330B is configured as a concave shape with the curved surface facing towards the inlet 316. The perforated bottom 330B serves to collect and isolate polyps and/or tissue samples removed during the endoscopy procedures. Its key function lies in filtration, with a fine mesh sieve allowing fluids to drain through while retaining solid tissue samples for examination. This mechanism ensures that the collected material remains intact and easily accessible for further analysis post-procedure, facilitating the identification and evaluation of tissue specimens in a controlled and organized manner.

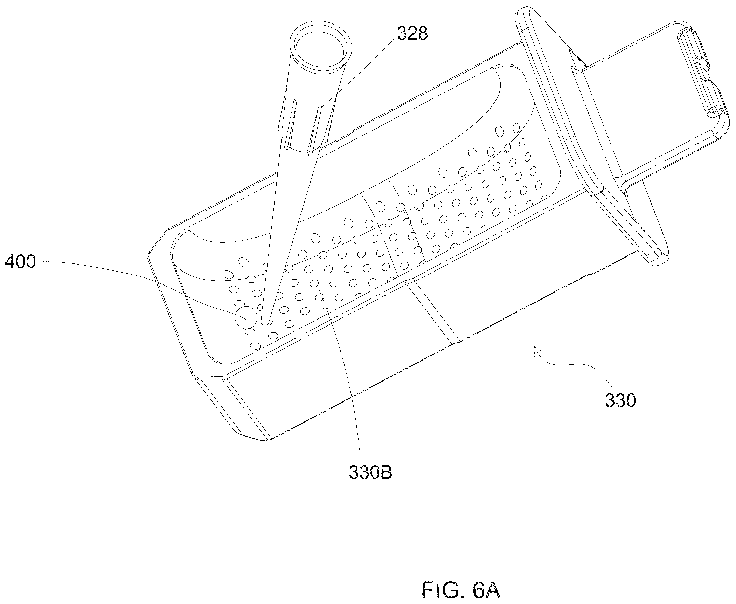

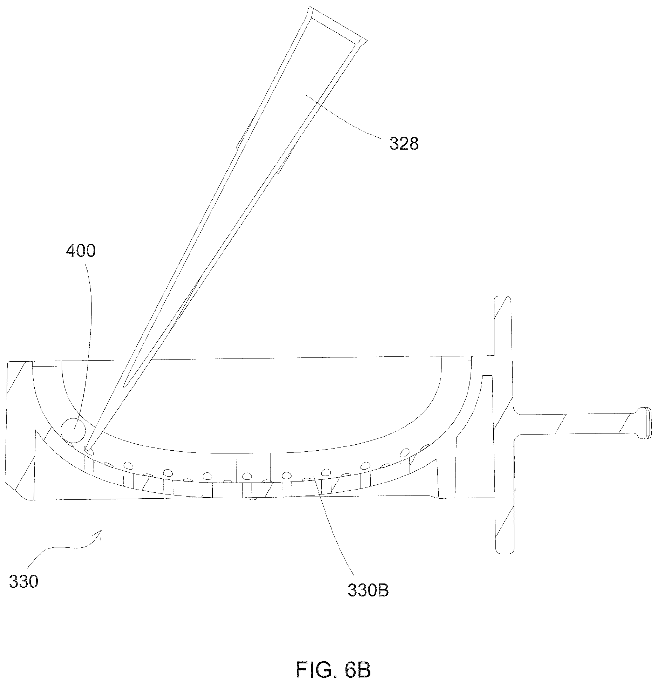

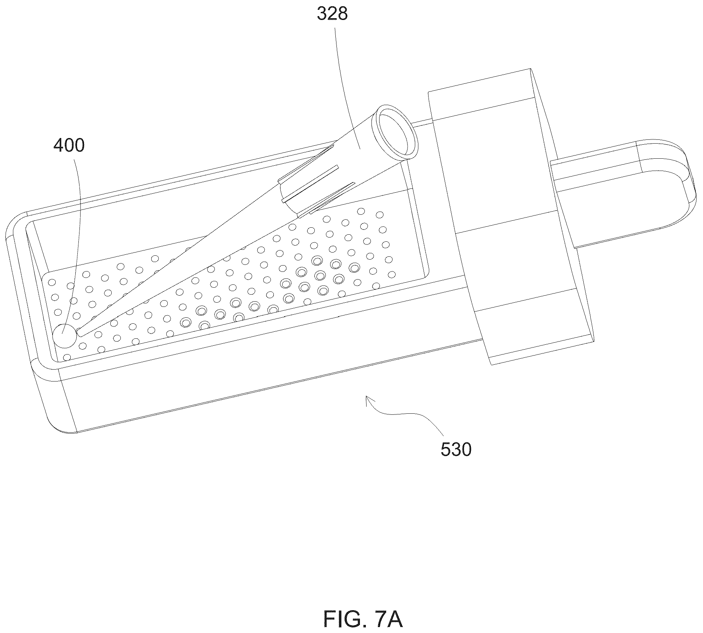

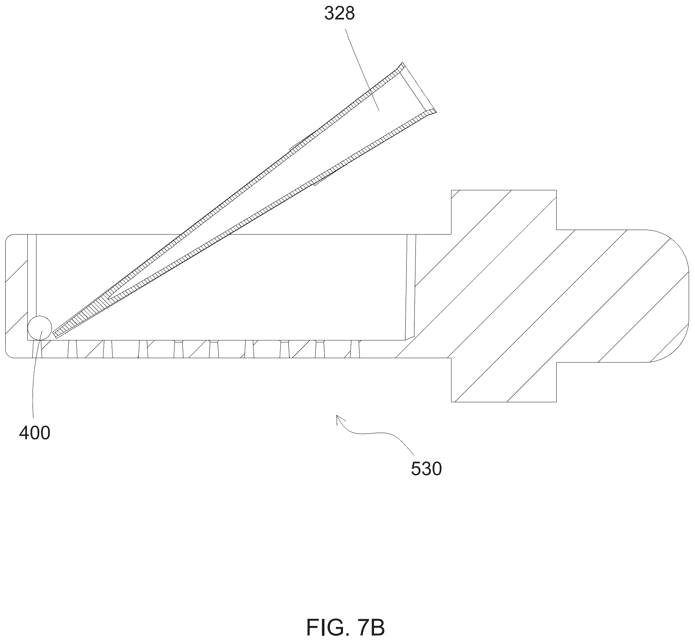

Figures 6A-6B illustrate how the curved surface aids in collecting and removing polyps or tissue samples. In this setup, the polyp or tissue sample 400 is gathered at the perforated bottom 330B. Medical personnel utilize the pick 328 to delicately remove the sample from this surface. The curved profile on the edge of the perforated bottom 330B is instrumental in this process, making it easier to remove the polyp or tissue sample 400 particularly at the corner without causing any damage or rupture, a risk that exists when dealing with sharp edges of a receptacle 530, as depicted in figures 7A-7B. This design not only ensures the integrity of the collected samples but also streamlines the removal process, contributing to the overall efficiency and safety of endoscopy procedures.

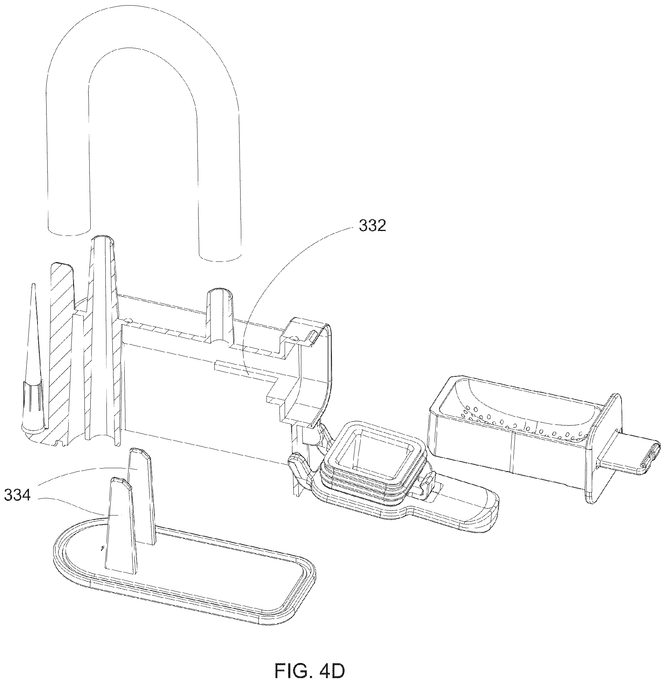

Figure 4D illustrates the interior of the main body 312 that is designed to provide a guiding system 332 to assist the smooth sliding and resting/positioning of the tray 330 inside the main body 312. The guiding system 332 can be a pair of tracks, rails, grooves, or a combination thereof. In some embodiments, the peripheral of the circumferential wall 330A of the tray 330 is provided with element(s) corresponding to the guiding system 332. For example, when the guiding system 332 is a pair of grooves, the peripheral of the circumferential wall 330A of the tray may be provided with a pair of ridges movably sliding inside the pair of grooves. This example is for illustration only and shall not be construed as any limitation to the present invention.

Figure 4D is an exploded view showing the polyp trap 310 and is a cross-sectional view showing the main body 312. It shows that the polyp trap 310 further comprises at least one stopper 334 positioned in the interior of the main body 312. The stopper 334 extends/stands upwards from the bottom of the main body 312, which collaborates with the guiding system 332 to position the tray 330 inside the main body 312. Additionally, the stopper 334 serves a second function of supporting the tray 330. In certain embodiments, the stopper 334 is shown standing upwards from the bottom within the interior of the main body 312. It is important to note that this specific orientation is provided for illustrative purposes only and should not be regarded as a limitation of the present invention. Skilled individuals in the art would have the capacity to make reasonable alterations; for instance, by configuring the stopper to extend downwards from the top within the main body 312. As long as the stopper is configured to restrict the sliding movement of the tray and/or provide support to the tray, it falls well within the scope of the present invention. In some embodiments, the number of the stopper can be two, three, four, or any integer.

The above-described shall not be interpreted to be restricted by the examples or figures only. It is to be expressly understood, however, that such modifications and adaptations are within the scope of invention in this aspect. For instance, features illustrated or described as part of one embodiment can be used on another embodiment to yield a still further embodiment. Thus, it is intended that the present disclosure cover such modifications and variations and their equivalents.

### List of reference numerals

- 100: set-up
- 102: suction pump
- 104: collection tank
- 106: light source
- 108: endoscope
- 108A: insertion section
- 108B: control section
- 108C: connector section
- 110: polyp trap
- 110A: inlet
- 110B: outlet
- 110C: tissue fluid or liquid
- 110D: door
- 202: tube
- 204: tube
- 300: set-up
- 310: polyp trap
- 312: main body
- 314: door
- 314A: circumferential wall
- 314B: sealing ridge
- 314C: first end
- 314D: handle
- 314E: tongue
- 314F: protrusion
- 316: inlet
- 318: outlet
- 320: first stand
- 322: tube
- 322A: first end
- 322B: second end
- 323: tube
- 324: opening
- 326: second stand
- 328: pick
- 330: tray
- 330A: circumferential wall
- 330B: perforated bottom
- 330C: handle
- 332: guiding system
- 334: stopper

## Claims

1. A polyp trap, configurable to connect to an endoscope and a vacuum system in endoscopy procedures, comprising:
- a hollow main body having an inlet and an outlet, wherein said main body, said inlet, and said outlet are fluidically communicable; and
- a tube having a first end removably connectable to said inlet and a second end removably connectable to the endoscope,
**characterized in that**,
said polyp trap further comprising a first stand positioned peripherally to said main body for receiving said second end of said tube, whereby said tube can selectively connect to said first stand when said polyp trap is in packaging or connect to the endoscope when said polyp trap is in the endoscopy procedures.

2. The polyp trap as claimed in claim 1, **characterized in that** said polyp trap further comprising:
- at least one second stand positioned peripherally to said main body, and at least one pick removably received by said second stand; and/or
- a tray received in said main body and designed as a receptacle having a circumferential wall and a perforated bottom, wherein said perforated bottom being configured as a concave shape with the curved surface facing towards said inlet; and/or
- at least one stopper standing inside said main body.

3. The polyp trap as claimed in claim 1, **characterized in that** said main body is made of transparent material.

4. The polyp trap as claimed in claim 2, **characterized in that** said main body is made of transparent material.

5. The polyp trap as claimed in claim 1 or 3, **characterized in that**:
- said main body comprising an opening providing access to the internal cavity of said main body; and
- said polyp trap comprising a door having a first end hingedly connected to said main body and a second end profiled as a handle, whereby said door is configured to open and close said opening with said handle by flipping down vertically and flipping up vertically, respectively.

6. The polyp trap as claimed in claim 2 or 4, **characterized in that**:
- said main body comprising an opening providing access to the internal cavity of said main body; and
- said polyp trap comprising a door having a first end hingedly connected to said main body and a second end profiled as a handle, whereby said door is configured to open and close said opening with said handle by flipping down vertically and flipping up vertically, respectively.

7. The polyp trap as claimed in claim 5, **characterized in that** said door being provided with a circumferential wall on one of its surfaces, wherein said circumferential wall is sized and shaped to be fit snugly within said opening of said main body.

8. The polyp trap as claimed in claim 6, **characterized in that** said door being provided with a circumferential wall on one of its surfaces, wherein said circumferential wall is sized and shaped to be fit snugly within said opening of said main body.

9. The polyp trap as claimed in claim 7, **characterized in that** surrounding the perimeter of said circumferential wall is at least one sealing ridge for enhancing the sealing capability of said door when closing said opening.

10. The polyp trap as claimed in claim 8, **characterized in that** surrounding the perimeter of said circumferential wall is at least one sealing ridge for enhancing the sealing capability of said door when closing said opening.

11. The polyp trap as claimed in claim 9, **characterized in that** said sealing ridge is made of resilient material.

12. The polyp trap as claimed in claim 10, **characterized in that** said sealing ridge is made of resilient material.
